(19) Europäisches Patentamt
European Patent Office
Office européen des brevets

(11) Veröffentlichungsnummer: **0 001 090**
**A2**

(12) # EUROPÄISCHE PATENTANMELDUNG

(21) Anmeldenummer: **78100783.6**

(22) Anmeldetag: **30.08.78**

(51) Int. Cl.$^2$: **A 01 N 9/22**
A 01 N 9/12, C 07 D 253/08

(30) Priorität: **10.09.77 DE 2740887**

(43) Veröffentlichungstag der Anmeldung:
**21.03.79 Patentblatt 79/6**

(84) Benannte Vertragsstaaten:
**BE CH DE FR GB NL**

(71) Anmelder: **Bayer Aktiengesellschaft**
**Zentralbereich Patente, Marken und Lizenzen Bayerwerk**
**D-5090 Leverkusen 1(DE)**

(72) Erfinder: **Sasse, Klaus, Dr.**
**Puetzweg 13**
**D-5060 Bergisch-Gladbach 2(DE)**

(72) Erfinder: **Kraus, Peter, Dr.**
**Düsseldorfer Strasse 43**
**D-5000 Köln 80(DE)**

(72) Erfinder: **Scheinpflug, Hans, Dr.**
**Am Thelenhof 15**
**D-5090 Leverkusen(DE)**

(54) **Verfahren zur Herstellung von 3-Halogen-benzotriazin-1-oxiden und deren Verwendung zur Bekämpfung von Pflanzenbakteriosen.**

(57) Die Erfindung betrifft die Verwendung von teilweise bekannten 2-Halogen- benzotriazin-1- oxiden als Mittel zur Bekämpfung von Pflanzenbakteriosen.
Die Verbindungen der Formel

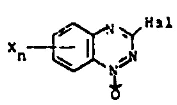

in welcher X, Hal und n die in der Beschreibung angegebene Bedeutung besitzen, weisen starke bakterizide Wirkungen auf. Sie können als Pflanzenschutzmittel Verwendung finden zur Behandlung oberirdischer Pflanzenteile, zur Saatgutbehandlung und zur Bodenbehandlung. Die erfindungsgemäß verwendbaren Stoffe sind but pflanzenverträglich.

EP 0 001 090 A2

BAYER AKTIENGESELLSCHAFT
Zentralbereich
Patente, Marken und Lizenzen

5090 Leverkusen, Bayerwerk
Slr-kl
IIb (IVa)

BEZEICHNUNG GEÄNDE
siehe Titelseite

Mittel zur Bekämpfung von Pflanzenbakteriosen

Die vorliegende Erfindung betrifft die Verwendung von teilweise bekannten 2-Halogen-benzotriazin-1-oxiden als Mittel zur Bekämpfung von Pflanzenbakteriosen. Sie betrifft ferner ein spezielles chemisches Verfahren zur Herstellung der Verbindungen.

Durch pathogene Bakterien verursachte Pflanzenkrankheiten breiten sich in zunehmendem Maße aus, so daß bereits jetzt die Anbauwürdigkeit einiger Kulturpflanzen in bestimmten Regionen wirtschaftlich gefährdet ist. Bedeutende Erreger entstammen der Familie der Pseudomonadaceae, z.B. Pseudomonas solanacearum, Pseudomonas lachrymans, Pseudomonas syringae, Xanthomonas citri, Xanthomonas oryzae, Xanthomonas vesicatoria, der Familie der Enterobacteriaceae, z.B. Erwinia amylovora, bzw. der Familie der Corynebacteriaceae. Die bisher zur Verfügung stehenden Möglichkeiten zur Bekämpfung bzw. Verhütung dieser Krankheiten sind sehr aufwendig und dabei noch unzureichend. Im praktischen Einsatz befinden sich hierfür fast ausschließlich Antibiotika, deren Herstellung teuer ist und die vielfach toxisch und

unter atmosphärischen Bedingungen nur kurze Zeit stabil sind.

Es ist bereits bekannt geworden, daß solche Benzotriazin-1-oxide, die in 3-Stellung Halogen, eine Amino-, Hydrazino-, Alkoxy- oder Alkylmercapto-Gruppe besitzen, herbizide, akarizide und fungizide Eigenschaften aufweisen (vgl. hierzu DL-PS 33 869); fungizid wirksam sind z.B. 3-Chlor-benzotriazin-1-oxid und 3,7-Dichlor-benzotriazin-1-oxid. Über bakterizide Eigenschaften ist jedoch nichts bekannt.

Es wurde nun gefunden, daß die 3-Halogen-benzotriazin-1-oxide der Formel

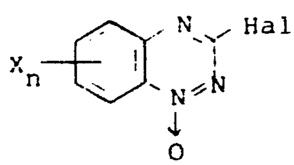

(I)

in welcher

X für Halogen, für die Nitro-Gruppe, für Alkyl-, Halogenalkyl-, Alkoxy-, Alkylmercapto- und/oder Alkylsulfonyl-Gruppen mit jeweils bis zu 4 Kohlenstoffatomen, für Phenoxy- und/oder Phenylmercapto-Reste steht, und

n für ganze Zahlen von 0 bis 4 steht, und

Hal für Chlor oder Brom steht,

eine hohe Wirksamkeit gegen pflanzenschädigende Bakterien besitzen.

Es ist als sehr überraschend zu bezeichnen, daß der obengenannte Verbindungstyp bakterizide Wirkungen entfaltet, da von keinem im Praxiseinsatz befindlichen Fungizid gleichzeitig auch praktisch nutzbare bakterizide Eigenschaften erkannt worden sind. Die Verwendung dieser Stoffe als Bakterizide für den Pflanzenschutz stellt daher eine Bereicherung der Technik dar; auch übertreffen diese Stoffe im Wirkungsgrad und in der Stabilität unter atmosphärischen Bedingungen die zur Zeit im Einsatz befindlichen Antibiotika.

Die erfindungsgemäß zu verwendenden 2-Halogen-benzotriazin-1-oxide sind durch die Formel I definiert. In der Formel I steht X vorzugsweise für Chlor oder Brom, für Methoxy-, Phenoxy-, Methylmercapto-, Methyl-, Trifluormethyl- und/oder Nitro-Gruppen. Der Index n steht vorzugsweise für die Zahlen 0, 1 und 2. Hal steht vorzugsweise für Chlor.

Als Beispiele für die erfindungsgemäßen Wirkstoffe seien im einzelnen genannt:

3-Chlor-benzotriazin-1-oxid
3-Brom-benzotriazin-1-oxid
3,5-Dichlor-benzotriazin-1-oxid
3,6-Dichlor-benzotriazin-1-oxid
3,7-Dichlor-benzotriazin-1-oxid
3-Brom-7-chlor-benzotriazin-1-oxid
3,8-Dichlor-benzotriazin-1-oxid

3,5,7-Trichlor-benzotriazin-1-oxid
3,6,7-Trichlor-benzotriazin-1-oxid
3,5,8-Trichlor-benzotriazin-1-oxid
3-Chlor-7-brom-benzotriazin-1-oxid
3-Chlor-5,7-dibrom-benzotriazin-1-oxid
3-Chlor-7-methoxy-benzotriazin-1-oxid
3-Chlor-7-phenoxy-benzotriazin-1-oxid
3-Chlor-7-methyl-benzotriazin-1-oxid
3-Chlor-7-methylmercapto-benzotriazin-1-oxid
3-Chlor-7-methylsulfonyl-benzotriazin-1-oxid
3-Chlor-7-trifluormethyl-benzotriazin-1-oxid
3-Chlor-7-nitro-benzotriazin-1-oxid

Die 3-Halogen-benzotriazin-1-oxide der Formel I sind ein grundsätzlich bekannter Verbindungstyp. So wird das 3-Chlor-benzotriazin-1-oxid selbst aus 3-Hydroxy-benzotriazin-1-oxid durch Umsetzung mit siedendem Phosphoroxychlorid gewonnen (J. Chem. Soc. 1957, 3186), und in entsprechender Weise wird mit Phosphoroxybromid das 3-Brom-benzotriazin-1-oxid erhalten (vgl. J. Org. Chem. 24, 813 (1959)). In analoger Weise wurden bereits 3,7-Dichlor-benzotriazin-1-oxid und 3-Chlor-7-methoxy-benzotriazin-1-oxid dargestellt (J. Org. Chem. 24, 813 (1959)). Dagegen sind 3-Chlor-benzotriazin-1-oxide mit mehreren Chloratomen im Phenylrest sowie mit Brom-, Nitro- oder Trifluormethylsubstituenten bisher nicht bekannt geworden. Das mag zum Teil darin begründet sein, daß sich die herfür erforderlichen Zwischenprodukte nicht ohne spezielle Maßnahmen auf üblichem Wege herstellen lassen.

Das aus der Literatur (Chem. Ber. 46, 3522 (1913); 50, 1248 (1917)) bekannte Verfahren zur Darstellung der 3-Hydroxy-benzotriazin-1-oxide besteht darin, daß ein evtl. zusätzlich substituiertes 2-Nitro-anilin (II) entweder mit Phosgen in das 2-Nitro-phenylisocyanat (III) und dieses mit Ammoniak in den 2-Nitro-phenylharnstoff (IV) übergeführt wird, den man anschließend mit Alkali zum 3-Hydroxy-benzotriazin-1-oxid (V) cyclisiert; oder daß die Verbindung II mit Cyanamid zum 2-Nitro-phenylguanidin (VI) umgesetzt, im alkalischen Medium zum 3-Amino-benzotriazin-1-oxid (VII) cyclisiert und dann mit salpetriger Säure zu (V) desaminiert wird:

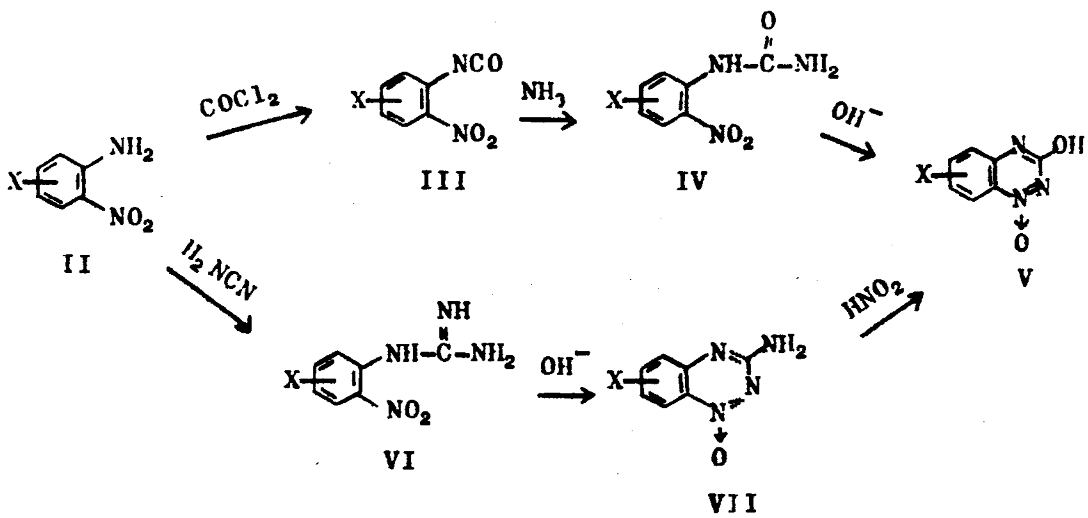


II, III, IV, V, VI, VII

Die Phosgenierung der 2-Nitro-aniline (II) zu den Isocyanaten (III) sowie auch deren Umsetzung mit Cyanamid zu den Guanidinen (VI) versagt jedoch unter den üblichen

Methoden, wenn z.B. mehr als ein weiterer Halogensubstituent oder z.B. eine weitere Nitrogruppe im Molekül (II) enthalten ist.

Zur Darstellung der nach der angegebenen Arbeitsweise nicht erhältlichen, als Zwischenprodukte benötigten Isocyanate der Formel (III) wurde daher die Phosgenierung des entsprechenden 2-Nitroanilins der Formel II in Phosphoroxychlorid als Reaktionsmedium durchgeführt, und nicht, wie sonst üblich, in Kohlenwasserstoffen. Die Folgeumsetzung über die Verbindungen der Formel IV zu den Verbindungen der Formel V gelingt dann in üblicher Weise.

Zur Darstellung von solchen als Ausgangsprodukten benötigten 3-Hydroxy-benzotriazin-1-oxiden, die eine Nitrogruppe in 7-Stellung besitzen (X steht in Formel V in diesem Falle für $NO_2$), wurde noch zusätzlich folgendes Verfahren gefunden: die Nitrierung des 3-Hydroxy-benzotriazin-1-oxids wird mit einem Salpetersäure/Schwefelsäure-Gemisch im Temperaturbereich zwischen 0 und 25°C durchgeführt:

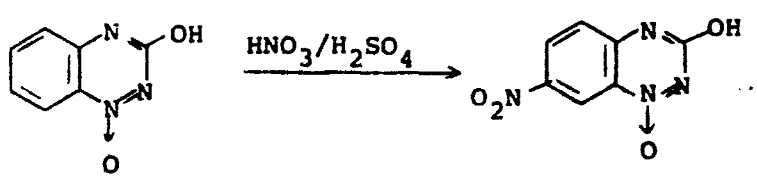


Es ist überraschend, daß diese Nitrierungsreaktion gelingt und in einfacher und einheitlicher Weise zur 7-Nitro-Verbindung führt, da nach Literaturangaben eine Nitrierung des 3-Hydroxy-benzotriazin-1-oxids nicht möglich sein sollte (J. Chem. Soc. 1957, 3186). Die Nitrie-

rung verläuft z.B. quantitativ, wenn man mit einer Mischung aus 28 % Salpetersäure (63 %ige Säure), 56 % konzentrierter Schwefelsäure und 16 % Wasser arbeitet (sogenannte "Mischsäure T").

Wie oben beschrieben, lassen sich die erfindungsgemäß verwendbaren 3-Halogen-benzotriazin-1-oxide der allgemeinen Formel I aus den entsprechenden 3-Hydroxy-Verbindungen der Formel V und siedendem Phosphoroxyhalogenid herstellen. Die Reaktion verläuft jedoch in manchen Fällen unbefriedigend. Auch bei verlängerter Reaktionszeit oder anderer Abwandlung der bekannten Reaktionsbedingungen entstehen meist größere Mengen von in organischen Lösungsmitteln unlöslichen Nebenprodukten. Auch Zusätze von bis zu molaren Mengen an Phosphor-V-halogenid erbringen keine besseren Ausbeuten.

Es wurde nun weiterhin gefunden, daß man die erfindungsgemäß verwendbaren, teilweise bekannten 3-Halogen-benzotriazin-1-oxide der allgemeinen Formel I vorteilhaft dann herstellen kann, wenn man die 3-Hydroxy-benzotriazin-1-oxide der Formel

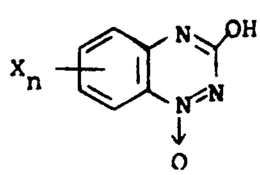

(V)

in welcher
X und n die oben angegebene Bedeutung besitzen,

mit Thionylhalogenid der Formel

$$SO(Hal)_2 \qquad (VI)$$

in welcher
Hal die weiter oben angegebene Bedeutung besitzt

in Gegenwart von Carbonsäureamiden im Temperaturbereich zwischen +50 und 180°C umsetzt.

Das Verfahren, das sich insbesondere zur Herstellung von 3-Chlor-Verbindungen eignet (Hal steht somit bevorzugt für Chlor), weist technische Vorteile auf: Außer der Vermeidung unlöslicher Nebenprodukte und der Steigerung der Ausbeute hat es den Vorteil, daß eine wäßrige Aufarbeitung zur Isolierung der Verbindungen der Formel I nicht erforderlich ist. Bei der bekannten Verwendung von Phosphoroxyhalogenid als Reaktionspartner entstehen zwangsläufig Polyphosphorsäurehalogenide als Beiprodukte, die durch Zerlegung mit Wasser in (Poly-)Phosphorsäuren übergeführt werden müssen. Beim Arbeiten mit Thionylhalogenid, aus dem während der Reaktion Schwefeldioxid und Halogenwasserstoff entstehen, genügt es, den Überschuß an Thionylhalogenid abzudestillieren. Die Abtrennung der Reaktionsprodukte I von den aus dem Katalysator (z.B. Dimethylformamid) mit Thionylhalogenid entstehenden salzartigen Verbindungen gelingt in einfacher Weise durch Extraktion mit organischen Lösungsmitteln, wie aliphatischen und aromatischen Kohlenwasserstoffen, Chlorkohlenwasserstoffen, Essigester und dergleichen.

Verwendet man 3-Hydroxy-7-chlor-benzotriazin-1-oxid und Thionylchlorid als Ausgangsstoffe, so kann der Reaktionsablauf durch das folgende Formelschema wiedergegeben werden:

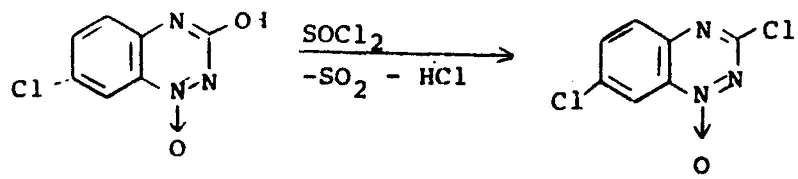


Die erfindungsgemäße Umsetzung wird in Gegenwart von Carbonsäureamiden als Katalysatoren vorgenommen. Im Gegensatz zu der bekannten Methode, nach der sich verschiedene Hydroxy-N-heterocyclen auch durch Thionylchlorid in Chlor-N-heterocyclen umwandeln lassen, wird in den Fällen, in welchen die 3-Hydroxy-benzotriazin-1-oxide der Formel V im Phenylrest unsubstituiert oder monochlorsubstituiert sind, anders verfahren: Es werden mindestens äquimolare Mengen des Katalysators, vorzugsweise Dimethylformamid, allmählich in das wenigstens 2 Mol Thionylhalogenid pro Mol Hydroxy-Verbindung enthaltende Reaktionsgemisch zugegeben. Es ist überraschend, daß bei dieser Arbeitsweise hohe Ausbeuten erzielt werden, während bei der bekannten Methode (Einsatz von nur 0,01 bis 0,1 Mol Dimethylformamid) nur geringfügige Umsetzungen festgestellt werden können. Es war weiterhin überraschend festzustellen, daß die bei andersartigen Umsetzungen üblicherweise aufgewendeten Mengen von 1 bis 20 Molprozent an Katalysator dann ausreichen, wenn man von solchen 3-Hydroxy-benzotriazin-1-oxiden

der Formel V ausgeht, die entweder mehrere Chloratome oder mindestens ein Bromatom oder eine Trifluormethylgruppe oder eine Nitrogruppe im Phenylrest als Substituenten enthalten.

Das für die erfindungsgemäße Reaktion benötigte Thionylhalogenid wird im Überschuß verwendet; man setzt 2 bis 30 Mol, vorzugsweise 2 bis 15 Mol, Thionylhalogenid, vorzugsweise Thionylchlorid, pro Mol der Hydroxy-Verbindung der Formel V ein. Das Thionylhalogenid dient auch gleichzeitig als Lösungsmittel. Es können jedoch noch weitere Lösungsmittel mit einem Siedepunkt von oberhalb +60°C, wie z.B. Tetrachlormethan, Toluol, Nitrobenzol, Chlorbenzol und dergleichen als Verdünnungsmittel zugesetzt werden.

Die Umsetzung wird im Temperaturbereich zwischen +50 und 180°C, vorzugsweise zwischen 75 und 150°C, durchgeführt.

Nach dem erfindungsgemäßen Verfahren können auch die folgenden neuen Stoffe hergestellt werden:

3,6-Dichlor-benzotriazin-1-oxid
3,5,7-Trichlor-benzotriazin-1-oxid
3,6,7-Trichlor-benzotriazin-1-oxid
3-Chlor-7-brom-benzotriazin-1-oxid
3-Chlor-7-phenoxy-benzotriazin-1-oxid
3-Chlor-7-trifluormethyl-benzotriazin-1-oxid
3-Chlor-7-nitro-benzotriazin-1-oxid

Nähere Angaben hierzu finden sich bei den Herstellungsbeispielen.

Die erfindungsgemäß verwendbaren Wirkstoffe weisen starke bakterizide Wirkungen auf. Sie können als Pflanzenschutzmittel Verwendung finden, zur Behandlung oberirdischer Pflanzenteile, zur Saatgutbehandlung und zur Bodenbehandlung.

Bakterizide Mittel werden im Pflanzenschutz eingesetzt zur Bekämpfung von Bakterien aus der Familie der Pseudomonadaceae, z.B. von Pseudomonas solanacearum, Pseudomonas lachrymans, Pseudomonas syringae, Xanthomonas citri, Xanthomonas oryzae und Xanthomonas vesicatoria, der Familie der Enterobacteriaceae, z.B. von Erwinia amylovora, bzw. der Familie der Corynebacteriaceae, ferner aus der Familie der Rhizobiaceae, z.B. von Agrobacterium tumefaciens.

Die erfindungsgemäß verwendbaren Stoffe sind gut pflanzenverträglich.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattbakterizide können die Wirkstoffkonzentrationen in den Anwendungsformen in einem größeren Bereich variiert werden. Sie liegen im allgemeinen zwischen 0,5 und 0,0005 Gewichtsprozenten, vorzugsweise zwischen 0,2 und 0,001 %.

Bei der Saatgutbehandlung werden im allgemeinen Wirkstoffmengen von 0,01 bis 50 g je Kilogramm Saatgut, vorzugsweise 0,5 bis 5 g, benötigt.

Zur Bodenbehandlung sind Wirkstoffmengen von 1 bis 1000 g je cbm Boden, vorzugsweise von 10 bis 200 g, erforderlich.

Die Verwendungsmöglichkeiten gehen aus den nachfolgenden Beispielen hervor:

Beispiel A

Agarplatten-Test

Verwendeter Nährboden:

20 Gew.-Teile Agar-Agar
200 Gew.-Teile Kartoffeldekokt
5 Gew.-Teile Malz
15 Gew.-Teile Dextrose
5 Gew.-Teile Pepton
2 Gew.-Teile Dinatrium-hydrogenphosphat
0,3 Gew.-Teile Calciumnitrat

Verhältnis von Lösungsmittelgemisch zum Nährboden:

2 Gew.-Teile Lösungsmittelgemisch
100 Gew.-Teile Agarnährboden

Zusammensetzung Lösungsmittelgemisch:

| | | |
|---|---|---|
| 0,19 | Gew.-Teile | Dimethylformamid oder Aceton |
| 0,01 | Gew.-Teile | Emulgator Alkylarylpolyglykoläther |
| 1,80 | Gew.-Teile | Wasser |
| 2 | Gew.-Teile | Lösungsmittelgemisch |

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden

Kontrollplatten ohne Präparatbeimischung aufgestellt.

Ist der Nährboden erkaltet und fest, werden die Platten mit den in der Tabelle angegebenen Bakterien beimpft und bei etwa 21°C inkubiert.

Die Auswertung erfolgt je nach der Wachstumsgeschwindigkeit der Bakterien nach 2 bis 4 Tagen. Bei der Auswertung wird das Wachstum auf den behandelten Nährboden mit dem Wachstum auf dem Kontrollnährboden verglichen. Die Bonitierung des Wachstums geschieht mit folgenden Kennzahlen:

| | |
|---|---|
| 1 | kein Wachstum |
| bis 3 | sehr starke Hemmung des Wachstums |
| bis 5 | mittelstarke Hemmung des Wachstums |
| bis 7 | schwache Hemmung des Wachstums |
| 9 | Wachstum gleich der unbehandelten Kontrolle |

Wirkstoffe, Wirkstoffkonzentration und Resultate gehen aus der nachfolgenden Tabelle hervor:

Tabelle A

Agarplatten-Test

| Wirkstoffe | Wirkstoffkonz. [ppm] | Pseudomonas lachrymans | Xanthomonas begoniae | Xanthomonas pelargonii | Erwinia carotovora | Erwinia mangiferae | Xanthomonas vesicatoria |
|---|---|---|---|---|---|---|---|
| Kupferoxychlorid<br>3 $Cu(OH)_2 \cdot CuCl_2 \cdot xH_2O$<br>(bekannt) | 50 | 9 | 5 | 5 | 5 | 3 | 9 |
| 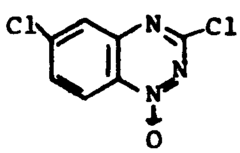 | 50 | - | 2 | 3 | 1 | - | - |
| 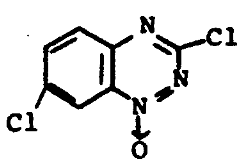 | 50 | 1 | 1 | 1 | 1 | 1 | 2 |
| 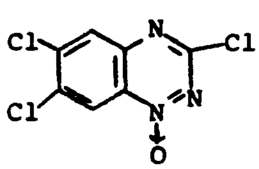 | 50 | 2 | 1 | 2 | 1 | - | 5 |
| 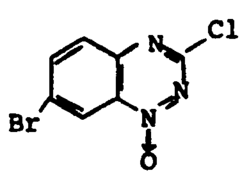 | 50 | 1 | 1 | 1 | 1 | - | 5 |

Beispiel B

Bakterien-Test / Xanthomonas oryzae

| | | |
|---|---|---|
| Lösungsmittel: | 11,75 | Gew.-Teile Aceton |
| Dispergiermittel: | 0,75 | Gew.-Teile Alkylarylpolyglykoläther |
| Wasser: | 987,50 | Gew.-Teile |
| andere Zusätze | - | Gew.-Teile - |

Man vermischt die für die gewünschte Wirkstoffkonzentration in der Spritzflüssigkeit nötige Wirkstoffmenge mit der angegebenen Menge des Lösungsmittels und des Dispergiermittels und verdünnt das Konzentrat mit der angegebenen Menge Wasser.

Mit der Spritzflüssigkeit bespritzt man 30 etwa 40 Tage alte Reispflanzen bis zur Tropfnässe. Die Pflanzen verbleiben bis zum Abtrocknen in einem Gewächshaus bei Temperaturen von 22 bis 24°C und einer relativen Luftfeuchtigkeit von etwa 70 %. Danach werden Nadeln in eine wäßrige Bakteriensuspension von Xanthomonas eryzae getaucht und die Pflanzen durch Anstechen der Blätter inokuliert. Die Pflanzen stehen nach der Inokulation 24 Stunden bei 100 % relativer Luftfeuchtigkeit und danach in einem Raum bei 26 bis 28°C und 80 % relativer Luftfeuchtigkeit. 10 Tage nach der Inokulation wird der Befall bei allen durch Stich verletzten, inokulierten Blätter von vorher mit Präparat behandelten Pflanzen in Wertzahlen von 1 bis 9 ausgewertet. 1 bedeutet 100 %ige Wirkung, 3 = gute Wirkung, 5 = mäßige Wirkung und 9 = keine Wirkung.

Wirkstoffe, Wirkstoffkonzentrationen und Resultate gehen aus der nachfolgenden Tabelle hervor:

# Tabelle B

Bakterien-Test / Xanthomonas oryzae

| Wirkstoff | Befallswertzahl bei einer Wirkstoffkonzentration (in %) von 0,025 | 0,05 |
|---|---|---|
| Kupferoxychlorid<br>3 $Cu(OH)_2 \cdot CuCl_2 \cdot xH_2O$<br>(bekannt) | 7 | 5 |
| 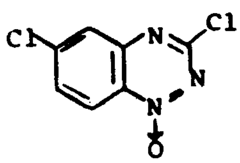 | - | 3 |
| 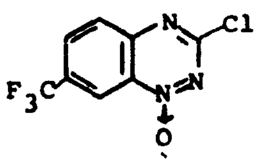 | - | 3 |
| 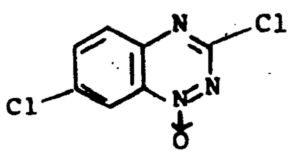 | 3 | 3 |
| 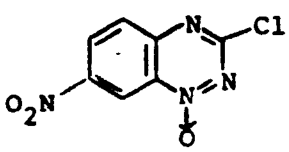 | - | 3 |
| 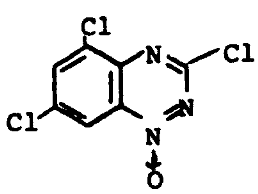 | 3 | - |

## Herstellungsbeispiele

### Beispiel 1

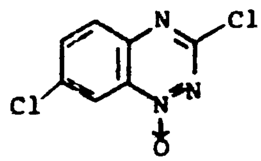

197,5 g (1 Mol) 3-Hydroxy-7-chlor-benzotriazin-1-oxid werden portionsweise in 1 l (ca. 1655 g oder 14 Mol) Thionylchlorid eingetragen. Man erhitzt das Gemisch zum Sieden und tropft innerhalb von 6 Stunden 116 ml (1,5 Mol) Dimethylformamid ein. Nachdem eine klare Lösung entstanden ist, hält man das Gemisch noch weitere 2 Stunden am Sieden und destilliert anschließend das überschüssige Thionylchlorid ab. Der Rückstand wird mit 1 l Toluol bei 60 bis 80°C verrührt. Man filtriert die unlöslichen Bestandteile ab und dampft das Toluol im Vakuum ab. Als Rückstand verbleiben 175 g (81 % der Theorie) 3,7-Dichlor-benzotriazin-1-oxid vom Fp. 157 - 158°C (aus Waschbenzin umgelöst).

In entsprechender Weise werden die folgenden Verbindungen der allgemeinen Formel

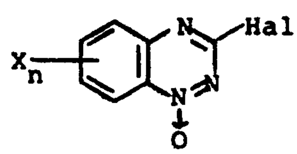

(I)

hergestellt:

| Beispiel-Nr. | $X_n$ | Hal | Ausbeute (% d.Th.) | Fp. |
|---|---|---|---|---|
| 2 | 6-Cl | Cl | 78 | 159-161 |
| 3 | 7-$CH_3O$ | Cl | 84 | 164-165 |
| 4 | 7- 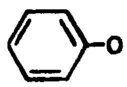 | Cl | 57 | 182-183 |
| 5 | 5,7-$Cl_2$ | Cl | 92 | 129-130 |
| 6 | 6,7-$Cl_2$ | Cl | 80 | 112-113 |

Beispiel 7

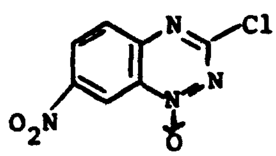

208 g (1 Mol) 3-Hydroxy-7-nitro-benzotriazin-1-oxid werden portionsweise in 1400 ml (2320 g oder 19,5 Mol) Thionylchlorid eingetragen. Dann setzt man 15 ml (0,2 Mol) Dimethylformamid zu und erhitzt das Gemsich zum Sieden, bis eine klare Lösung entstanden ist (etwa 4 bis 6 Stunden). Die Aufarbeitung erfolgt wie in Beispiel 1. Man erhält 195 g (86 % der Theorie) 3-Chlor-7-nitro-benzotriazin-1-oxid vom Fp. 174-175°C (aus Toluol umgelöst).

In entsprechender Weise werden die folgenden Verbindungen der allgemeinen Formel I hergestellt:

| Beispiel-Nr. | $X_n$ | Hal | Ausbeute (% d.Th.) | Fp. (°C) |
|---|---|---|---|---|
| 8 | 7-$CF_3$ | Cl | 83 | 108-109 |
| 9 | 7-Br | Cl | 78 | 150-152 |

Herstellung der Vorprodukte

2-Nitro-phenylharnstoffe der Formel

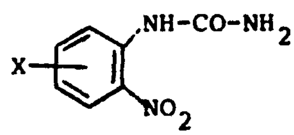

(IV)

Allgemeine Arbeitsweise:

In 1200 ml Phosphoroxychlorid, dem 5 ml Dimethylformamid zugefügt wurden, wird bei einer Temperatur von 0 bis 5°C 6 Stunden lang Phosgen einkondensiert. Bei der gleichen Temperatur trägt man dann portionsweise 1 Mol eines 2-Nitroanilins ein. Unter weiterem Einleiten von Phosgen erwärmt man das Gemisch innerhalb von 3 Stunden zum Sieden und kocht noch 3 Stunden unter Rückfluß. Dann wird das Phosphoroxychlorid im Vakuum abdestilliert. Das zurückbleibende rohe 2-Nitro-phenylisocyanat wird in 1 l Toluol gelöst, unlöslich verbleibende Anteile werden abfiltriert. In diese Lösung leitet man bei 20 bis 25°C gasförmiges Ammoniak bis zum Überschuß ein. Der dabei ausfallende 2-Nitro-phenylharnstoff wird abgesaugt, zur Entfernung salzartiger Beimengungen mit Wasser gewaschen und getrocknet.

Auf diesem Wege gewinnt man:

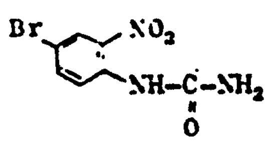

2-Nitro-4-brom-phenylharnstoff
Ausbeute: 88,5 %

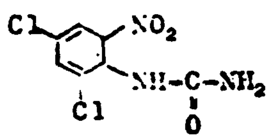

2-Nitro-4,6-dichlor-phenylharnstoff
Ausbeute: 93 %

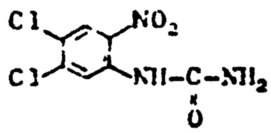

2-Nitro-4,5-dichlor-phenylharnstoff
Ausbeute: 66 %

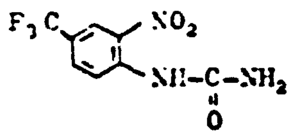

2-Nitro-4-trifluormethyl-phenylharnstoff
Ausbeute: 91 %

3-Hydroxy-benzotriazin-1-oxide der Formel

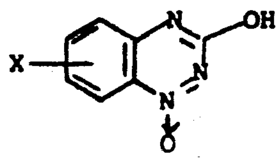

(V)

Allgemeine Arbeitsweise:

1 Mol eines 2-Nitro-phenylharnstoffes wird portionsweise in eine 70°C warme Lösung von 520 g NaOH in 1,3 l Wasser eingetragen. Das Gemisch wird während 2 Stunden bei 70°C gerührt, auf 50 bis 60°C abgekühlt und dann mit Salzsäure angesäuert. Man kühlt weiter auf Raumtemperatur ab, saugt das Reaktionsprodukt ab, wäscht es mehrfach mit Wasser und trocknet.

Auf diese Weise erhält man die folgenden Verbindungen der Formel V:

| X | Ausbeute |
|---|---|
| H | 97 % |
| 6-Cl | 81 % |
| 7-Cl | 97 % |

| X | Ausbeute |
|---|---|
| 5,7-Di-Cl | 88 % |
| 6,7-Di-Cl | 86 % |
| 7-Br | 95 % |
| 7-$OCH_3$ | 76 % |
| 7-O-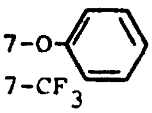 | 65 % |
| 7-$CF_3$ | 82 % |

3-Hydroxy-7-nitro-benzotriazin-1-oxid:

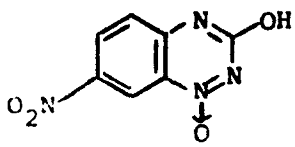

In 1,3 l einer Mischung aus 28 % konzentrierter Salpetersäure (63 %ig), 56 % konzentrierter Schwefelsäure und 16 % Wasser trägt man bei 0 bis 5$^{O}$C unter kräftigem Rühren portionsweise 163 g (1 Mol) 3-Hydroxy-benzotriazin-1-oxid ein. Man rührt 1 Stunde unter Eiskühlung nach, läßt die Temperatur innerhalb 2 Stunden auf Raumtemperatur ansteigen und rührt noch 2 Stunden bei Raumtemperatur nach. Dann rührt man das Reaktionsgemisch in 5 l Eiswasser ein, saugt die sich dabei abscheidenden Kristalle ab und wäscht sie mehrfach mit Wasser und trocknet. Ausbeute: praktisch quantitativ. Fp. 214$^{O}$C.

Patentansprüche

1. Bakterizide Mittel, gekennzeichnet durch einen Gehalt an mindestens einem 3-Halogen-benzotriazin-1-oxid der Formel

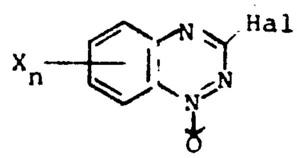

(I)

in welcher

X für Halogen, für die Nitro-Gruppe, für Alkyl-, Halogenalkyl-, Alkoxy-, Alkylmercapto- und/oder Alkylsulfonyl-Gruppen mit jeweils bis zu 4 Kohlenstoffatomen, für Phenoxy- und/oder Phenylmercapto-Reste steht, und

n für ganze Zahlen von 0 bis 4 steht, und

Hal für Chlor oder Brom steht.

2. Verfahren zur Bekämpfung von Bakterien, dadurch gekennzeichnet, daß man 3-Halogen-benzotriazin-1-oxide gemäß Anspruch 1 auf Bakterien oder ihren Lebenraum einwirken läßt.

3. Verwendung von 3-Halogen-benzotriazin-1-oxiden gemäß Anspruch 1 zur Bekämfpung von Bakterien.

4. Verfahren zur Herstellung von bakteriziden Mitteln, dadurch gekennzeichnet, daß man 3-Halogen-benzotriazin-1-oxide gemäß Anspruch 1 mit Streckmitteln und/oder oberflächenaktiven Mitteln vermischt.

5. Verfahren zur Herstellung von 3-Halogen-benzotriazin-1-oxiden, dadurch gekennzeichnet, daß man 3-Hydroxy-benzotriazin-1-oxide der Formel

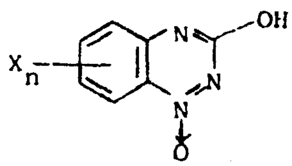

(V)

in welcher

X für Halogen, für die Nitro-Gruppe, für Alkyl-, Halogenalkyl-, Alkoxy-, Alkylmercapto- und/oder Alkylsulfonyl-Gruppen mit jeweils bis zu 4 Kohlenstoffatomen, für Phenoxy- und/oder Phenylmercapto-Reste steht, und

n für ganze Zahlen von 0 bis 4 steht,

mit Thionylhalogenid der Formel

$$SO\,(Hal)_2 \qquad (VI)$$

in welcher

Hal für Chlor oder Brom steht,

in Gegenwart von Carbonsäureamiden im Temperaturbereich zwischen 50 und 180°C umsetzt.

6. Verfahren gemäß Anspruch 5, dadurch gekennzeichnet, daß die Umsetzung in Gegenwart von Dimethylformamid durchgeführt wird.